# EUROPEAN PATENT SPECIFICATION

(11) **EP 2 685 840 B1**
(45) Date of publication and mention of the grant of the patent: **18.05.2016**
(21) Application number: 12711815.6
(22) Date of filing: 14.03.2012
(51) Int. Cl.: A23L 27/20, A23L 27/00, C07D 213/50

(54) **OFF-NOTE MASKING**
OFF-NOTE-MASKIERUNG
MASQUAGE D'UNE NOTE ATYPIQUE

(30) Priority: 14.03.2011 US 201161452456 P
(43) Date of publication of application: 22.01.2014
(73) Proprietor: Givaudan SA, 1214 Vernier (CH)
(72) Inventor: GRAY, Kimberley, Loveland Ohio 45140 (US); WANG, Yili, Mason Ohio 45040 (US); KELLER, Scott Wayne, Cincinnati Ohio 45208 (US)
(74) Representative: Givaudan Patents
(86) International application number: PCT/EP2012/054426
(87) International publication number: WO 2012/123475

(56) References cited:
- WO-A1-2011/004016
- US-A1- 2010 227 039
- US-A1- 2010 272 656
- US-A1- 2010 284 944

## Description

This disclosure relates to the masking of off-notes or off-tastes and to compounds and compositions for the masking of such off-notes or off-tastes.

Many orally-receivable or -ingestible compositions, by which is meant anything taken orally, either for ingestion or for spitting out, have off-tastes. Such off-tastes can be inherent to the composition itself, or they can be a result of a particular additive. Examples of compositions having an off-taste include coffee, soy milk and soy yoghurt. In the case of additives, particular examples are artificial sweeteners, which have been used in such compositions as a replacement for sugar and corn syrup for dietary or health reasons. These compounds, examples of which include saccharin, acesulfame-K and aspartame, are considerably sweeter than sugar, and so can be used in small amounts, replacing a considerably greater quantity of sugar. However, it has been noted that, although the predominant taste sensation of these compounds is sweetness, they frequently additionally provide an undesirable off-taste, often of a bitter or metallic quality, something absent from sugar and corn syrup. Such off-tastes limit the acceptance of compositions including such compounds, even though they are much healthier.

It has been proposed to add compounds that mask such off-tastes. A typical example of such a compound may be found in United States published application No. 2010/227039.

It has now been found that another compound has excellent off-note masking qualities for a wide range of off-tastes or off-notes. There is therefore provided a method of masking an off-note in an orally-receivable or -ingestible composition, comprising the addition thereto of an off-note masking quantity of 1-(2-hydroxyphenyl)-3-(pyridin-4-yl)propane-1-one or a salt thereof, and at least one of 4-[2,2,3-trimethyl-cyclopentyl]-butanoic acid or 2-[(3-ethyl-4,8-dimethyl-2-oxo-2H-chromen-7-yl)oxy]propanoic acid, or salts thereof.

There is also provided an orally-receivable or -ingestible composition comprising an off-note-masking proportion of 1-(2-hydroxyphenyl)-3-(pyridin-4-yl)propane-1-one or a salt thereof and at least one of 4-[2,2,3-trimethyl-cyclopentyl]-butanoic acid or 2-[(3-ethyl-4,8-dimethyl-2-oxo-2H-chromen-7-yl)oxy]propanoic acid, or salts thereof.

1-(2-hydroxyphenyl)-3-(pyridin-4-yl)propane-1-one (hereinafter referred to as "Compound 1") is disclosed in United States published application No. 2010/272656. In this publication, the compound is described as an effective sweetness enhancer. It is a surprising feature of this disclosure that the same compound can deliver an off-note- or off-taste-masking effect at a concentration far below the concentration at which it is perceived as sweet. The minimum level at which the sweetness enhancement effect of Compound 1 can be detected is 35 ppm, but the off-note- or off-taste-masking effect of the masker can be as low as 2.5 ppm. A practical maximum for off-note masking is 10 ppm.

***The*** off-note-masking effect of Compound 1 is enhanced yet further by the combination of certain other compounds with Compound 1. These are selected from 4-[2,2,3-trimethyl-cyclopentyl]-butanoic acid or 2-[(3-ethyl-4,8-dimethyl-2-oxo-2H-chromen-7-yl)oxy]propanoic acid, or salts thereof.

4-[2,2,3-trimethyl-cyclopentyl]-butanoic acid and 2-[(3-ethyl-4,8-dimethyl-2-oxo-2H-chromen-7-yl)oxy]propanoic acid (hereinafter respectively "Compound 2" and "Compound 3") are disclosed in United States Patent Publications US 2010/0284944 and US 2010/0227039 respectively.

Suitable salts of the Compounds 1, 2 or 3 may include but are not limited to at least one of K⁺, NH₄⁺, Na⁺, Ca²⁺, Mg²⁺, Al³⁺, Zn²⁺, hydrochloride or hydrobromide salt. Typical ratios of the compounds in masking compositions are now given. These are given by way of general indication only, and are not intended to be in any way limiting. A skilled person can create desirable effects by working outside these proportions.

Compound 1:Compound 2 ratio 1:40 up to 40:1, particularly 1:20 up to 20:1, more particularly 1:7 up to 6:1.

Compound 1:Compound 3 ratio 1:40 up to 40:1, particularly 1:20 up to 20:1, more particularly 1:7 up to 3:1.

Compound 1 and the masking composition may be used in all variety of orally-receivable and ingestible products. Non-limiting examples of such products include:
Consumable products, including, but not limited to all food products, food additives, nutraceuticals, pharmaceuticals and any product placed in the mouth including chewing gum, oral care products, and oral hygiene products including but not limited to, cereal products, rice products, tapioca products, sago products, baker's products, biscuit products, pastry products, bread products, confectionery products, dessert products, gums, chewing gums, mouthwash, dental floss, flavored or flavor-coated straws, flavor or flavor-coated food/beverage containers, chocolates, ices, honey products, treacle products, yeast products, baking-powder, salt and spice products, savoury products, mustard products, vinegar products, sauces (condiments), tobacco products, cigars, cigarettes, processed foods, cooked fruits and vegetable products, meat and meat products, jellies, jams, fruit sauces, egg products, milk and dairy products, yoghurts, cheese products, butter and butter substitute products, milk substitute products, soy products, edible oils and fat products, medicaments, beverages, carbonated beverages, alcoholic drinks such as beers, wines and spirits, non-alcoholic drinks such as soft drinks, mineral and aerated waters, fruit drinks, fruit juices, coffee, artificial coffee, tea, cocoa, including forms requiring reconstitution including, without limitation, beverage powder, milk based beverage powder, sugar-free beverage powder, beverage syrup, beverage concentrate, instant coffee, instant tea, instant cocoa, and coffee whitener, food extracts, plant extracts, meat extracts, condiments, gelatins, pharmaceutical and non-pharmaceutical gums, tablets, lozenges, drops, emulsions, elixirs, syrups and other preparations for making beverages, and combinations thereof.

Oral care products, including, but not limited to, any composition applied to the oral cavity for the purposes of cleaning, freshening, healing, deodorising the cavity or any part thereof, may include, but are not limited to, toothpastes, tooth gels, tooth powders, tooth whitening products, mouthwashes, lozenges, dental floss, toothpicks, anti-plaque and anti-gingivitis compositions, throat lozenges, throat drops, inflammatory compositions, compositions for treatment of nasal symptoms, cold symptoms and upper gastrointestinal tract distress, compositions for cold relief, for alleviating discomfort of hot flash, and gargle compositions.

Typical, non-limiting examples of Compound 1 are:
in a beverage 1 ppm to 20 ppm, particularly 5 ppm to 15 ppm,
in coffee between 1 ppm and 40 ppm, particularly 15 ppm to 30 ppm,
in soy milk between 1 ppm and 20 ppm, particularly 5 ppm to 15 ppm,
in mayonnaise between 1 ppm and 10 ppm, particularly 1 ppm to 5 ppm,
in OTC syrup between 15 ppm and 40 ppm, particularly 25 ppm to 35 ppm,
in proteinaceous material between 5 ppm and 40 ppm, particularly 20 ppm to 35 ppm.

The situation is more complex with mixtures of the Compounds 1, 2 and 3, and the following figures should be taken as indicative only, as the skilled person will certainly be able to find other possible combinations and possibilities:
Beverage;
   Compound 1:Compound 2 5ppm:35 ppm up to 15ppm:25ppm,
   Compound 1:Compound 3 ratio 5ppm:35ppm up to 15ppm:1ppm,
Coffee;
   Compound 1:Compound 2 ratio 15ppm:25ppm up to 25ppm:15ppm,
   Compound 1:Compound 3 ratio 15ppm:15ppm up to 25ppm:5ppm,
Soy milk;
   Compound 1:Compound 2 ratio 5ppm:15ppm up to 15ppm:5ppm,
   Compound 1:Compound 3 ratio 5ppm:15ppm up to 15ppm:5ppm,
Mayonnaise;
   Compound 1:Compound 2 ratio 1ppm:10ppm up to 5ppm:1ppm,
   Compound 1:Compound 3 ratio 1ppm:15ppm up to 5ppm:5ppm,
OTC syrup;
   Compound 1:Compound 2 ratio 25ppm:35ppm up to 35ppm:25ppm,
   Compound 1:Compound 3 ratio 25ppm:25ppm up to 35ppm:15ppm,
Proteinaceous material;
   Compound 1:Compound 2 ratio 20ppm:20ppm up to 35ppm:5ppm,
   Compound 1:Compound 3 ratio 20ppm:20ppm up to 35ppm:5ppm.

Compound 1 or masking composition may be used in such products with known flavor compounds, and also with all the known ancillary compounds used in flavoured products. They can be added and blended in the normal manner, like any other ingredient. In the case of the masking compositions, the individual components may be added separately, or preblended as a single additive.

The disclosure is further described with reference to the following examples, which describe certain embodiments, and which are not meant to be in any way limiting.

### Examples

The following compositions are made and tested blind. Using a blind bench-top tasting panel (consisting of 4 to 8 panelists), panelists were asked to record the sensory attribute differences between the control and example samples, specifically focusing on off-notes (reduction or differences).

The compounds are identified as follows:
Compound 1 = 1-(2-hydroxyphenyl)-3-(pyridin-4-yl)propane-1-one,
Compound 2 = 4-[2,2,3-trimethyl-cyclopentyl]-butanoic acid,
Compound 3 = 2-[(3-ethyl-4,8-dimethyl-2-oxo-2H-chromen-7-yl)oxy]propanoic acid.

All three compounds were used in the form of their hydrochloride salts.

### Example #1

### Full Sugar Energy Drink (Amp Energy™)

In this example, the drink used was Amp Energy™. This energy drink has the typical off-note characteristic of drinks based on caffeine, guarana and the like.

The proportions are as shown in Table 1.

**Table 1**

| Sample No. | Compound No. | Compound proportion (% by weight) | Drink proportion (% by weight) |
|---|---|---|---|
| 1 | - | - | 100 |
| 2 | 1 | 0.001 | to 100 |
| 3 | 2 | 0.001 | to 100 |
| 4 | 1/2 | 0.001/0.001 | to 100 |
| 5 | 3 | 0.0005 | to 100 |
| 6 | 1/3 | 0.001/0.0005 | to 100 |

*Bench taste findings:* Panelists detected significantly less off-note in Sample 4 (0.001% of each of Compounds 1 and 2) over either Sample 3 (Compound 2 alone) or Sample 1 (control, no addition).

Panelists detected significantly less off-note in either Sample 2 or Sample 5 over the control. In Sample 6, there was detected significantly less off-note over the control.

### Example #2

### Full Sugar Energy Drink (Red Bull™)

The procedure of Example 1 was repeated using the proportions shown in Table 2:

**Table 2**

| Sample No. | Compound No. | Compound proportion (% by weight) | Drink proportion (% by weight) |
|---|---|---|---|
| 7 | - | - | 100 |
| 8 | 1 | 0.001 | to 100 |
| 9 | 2 | 0.001 | to 100 |
| 10 | 1/2 | 0.001/0.001 | to 100 |
| 11 | 3 | 0.0005 | to 100 |
| 12 | 1/3 | 0.001/0.0005 | to 100 |

*Bench taste findings:* Panelists detected significantly less off-note in Sample 10 (0.001% of each of Compounds 1 and 2) over either Sample 8 (Compound 2 alone) or Sample 7 (control, no addition).

Panelists detected significantly less off-note in either Sample 8 or Sample 11 over the control. In Sample 12, there was detected significantly less off-note over the control.

### Example #3

### Diet Energy Drink

The drink used was Amp Energy™ - Sugar Free. This drinks contains the artificial sweeteners sucralose and acesulfame-K, which are noted for having metallic off-tastes.

The procedure was carried out as described in Example 1, using the proportions shown in Table 3.

**Table 3**

| Sample No. | Compound No. | Compound proportion (% by weight) | Drink proportion (% by weight) |
|---|---|---|---|
| 13 | - | - | 100 |
| 14 | 1 | 0.001 | to 100 |
| 15 | 2 | 0.001 | to 100 |
| 16 | 1/2 | 0.001/0.001 | to 100 |
| 17 | 3 | 0.0005 | to 100 |
| 18 | 1/3 | 0.001/0.0005 | to 100 |

*Bench taste findings:* Panelists preferred all the samples over the control. Sample 16 was preferred over Sample 15.

Panelists preferred Sample 18 to either Sample 14 or Sample 17.

### Example #4

### Diet Carbonated Soft Drink (CSD)

The drink used was Tab™, a saccharin-sweetened drink. Saccharin is known to contribute an off-taste.

The procedure was carried out as described in Example 1, using the proportions shown in Table 4.

**Table 4**

| Sample No. | Compound No. | Compound proportion (% by weight) | Drink proportion (% by weight) |
|---|---|---|---|
| 19 | - | - | 100 |
| 20 | 1 | 0.00025 | to 100 |
| 21 | 1 | 0.0005 | to 100 |
| 22 | 2 | 0.0005 | to 100 |
| 23 | 2 | 0.001 | to 100 |
| 24 | 3 | 0.0005 | to 100 |
| 25 | 3 | 0.001 | to 100 |
| 26 | 1/2 | 0.0005/0.0005 | to 100 |
| 27 | 1/3 | 0.0005/0.001 | to 100 |

*Bench taste findings:* Panelists preferred Samples 21, 22, 24, 25, 26 and 27 over the control Sample 19.

### Example #5

### Diet Carbonated Soft Drink (CSD)

The drink used was Pepsi Max™, a drink sweetened with aspartame and acesulfame-K.

The procedure was carried out as described in Example 1, using the proportions shown in Table 5.

**Table 5**

| Sample No. | Compound No. | Compound proportion (% by weight) | Drink proportion (% by weight) |
|---|---|---|---|
| 28 | - | - | 100 |
| 29 | 1 | 0.001 | to 100 |
| 30 | 2 | 0.001 | to 100 |
| 31 | 1/2 | 0.001/0.001 | to 100 |
| 32 | 3 | 0.0005 | to 100 |
| 33 | 1/3 | 0.001/0.0005 | to 100 |

*Bench taste findings:* The panelists preferred all the samples over the control. Panelists preferred Sample 31 over Sample 30, and Sample 33 over Sample 32.

### Example #6

### Diet Carbonated Soft Drink (CSD)

The drink was Zevia™, sweetened with Rebaudioside A.

The procedure was carried out as described in Example 1, using the proportions shown in Table 6.

**Table 6**

| Sample No. | Compound No. | Compound proportion (% by weight) | Drink proportion (% by weight) |
|---|---|---|---|
| 34 | - | - | 100 |
| 35 | 1 | 0.0005 | to 100 |
| 36 | 2 | 0.003 | to 100 |
| 37 | 1/2 | 0.0005/0.003 | to 100 |
| 38 | 3 | 0.003 | to 100 |
| 39 | 1/3 | 0.0005/0.0003 | to 100 |

*Blench taste findings:* Panelists preferred Sample 37 over Samples 35, 36 and 38.

### Example #7

### Coffee (unsweetened)

The procedure was carried out as described in Example 1, using the proportions shown in Table 7.

**Table 7**

| Sample No. | Compound No. | Compound proportion (% by weight) | Drink proportion (% by weight) |
|---|---|---|---|
| 40 | - | - | 100 |
| 41 | 1 | 0.002 | to 100 |
| 42 | 1 | 0.003 | to 100 |
| 43 | 2 | 0.002 | to 100 |
| 44 | 2 | 0.003 | to 100 |
| 45 | 3 | 0.001 | to 100 |
| 46 | 3 | 0.002 | to 100 |
| 47 | 1/2 | 0.002/0.003 | to 100 |
| 48 | 1/3 | 0.002/0.001 | to 100 |

*Bench taste findings:*
(i) The panelists preferred Samples 41, 44 and 45 over the control;
(ii) The panelists preferred Sample 47 over Sample 44;
(iii) The panelists preferred either Sample 41 or Sample 45.

### Example #8

### Plain Soymilk (Private Selection Organic Plain Soymilk - 7 g protein per 8 oz serving)

The procedure was carried out as described in Example 1, using the proportions shown in Table 8.

**Table 8**

| Sample No. | Compound No. | Compound proportion (% by weight) | Drink proportion (% by weight) |
|---|---|---|---|
| 49 | - | - | 100 |
| 50 | 1 | 0.001 | to 100 |
| 51 | 1 | 0.002 | to 100 |
| 52 | 2 | 0.001 | to 100 |
| 53 | 2 | 0.002 | to 100 |
| 54 | 3 | 0.0005 | to 100 |
| 55 | 3 | 0.001 | to 100 |
| 56 | 1/2 | 0.001/0.001 | to 100 |
| 57 | 1/3 | 0.001/0.001 | to 100 |

*Bench taste findings:*
(i) Panelists preferred Sample 50 over the control.
(ii) Panelists preferred Sample 52 over the control.
(iii) Panelists preferred Sample 54 over the control.
(iv) Panelists preferred the Sample 56 over Sample 52.
(v) Panelists preferred Sample 57 over Sample 50 or Sample 55 alone.

### Example #9

### Soy Yogurt (O'Soy Organic Soy Yogurt Strawberry Flavor - 5 g protein per 4 oz serving)

| Ingredient | % (by weight) |
|---|---|
| Soy Yogurt (O'Soy Organic Soy Yogurt Strawberry Flavor) | 100 |

| Ingredient | % (by weight) |
|---|---|
| Compound 1 | 0.001 |
| Soy Yogurt (O'Soy Organic Soy Yogurt Strawberry Flavor) | Balance to 100 |

| Ingredient | % (by weight) |
|---|---|
| Compound 1 | 0.002 |
| Soy Yogurt (O'Soy Organic Soy Yogurt Strawberry Flavor) | Balance to 100 |

*Bench taste findings:* Panelists preferred 0.002% of Compound 1 over the control. The off-notes in this product are typically associated with soy protein.

### Example #10

### Soy Yogurt (O'Soy Organic Soy Yogurt Strawberry Flavor - 5 g protein per 4 oz serving)

| Ingredient | % (by weight) |
|---|---|
| Soy Yogurt (O'Soy Organic Soy Yogurt Strawberry Flavor) | 100 |

| Ingredient | % (by weight) |
|---|---|
| Compound 2 | 0.001 |
| Soy Yogurt (O'Soy Organic Soy Yogurt Strawberry Flavor) | Balance to 100 |

| Ingredient | % (by weight) |
|---|---|
| Compound 2 | 0.002 |
| Soy Yogurt (O'Soy Organic Soy Yogurt Strawberry Flavor) | Balance to 100 |

*Bench taste findings:* Panelists preferred 0.002% of Compound 2 over the control. The off-notes in this product are typically associated with soy protein.

### Example #11

### Soy Yogurt (O'Soy Organic Soy Yogurt Strawberry Flavor - 5 g protein per 4 oz serving)

| Ingredient | % (by weight) |
|---|---|
| Soy Yogurt (O'Soy Organic Soy Yogurt Strawberry Flavor) | 100 |

| Ingredient | % (by weight) |
|---|---|
| Compound 3 | 0.0005 |
| Soy Yogurt (O'Soy Organic Soy Yogurt Strawberry Flavor) | Balance to 100 |

| Ingredient | % (by weight) |
|---|---|
| Compound 3 | 0.001 |
| Soy Yogurt (O'Soy Organic Soy Yogurt Strawberry Flavor) | Balance to 100 |

*Bench taste findings:* Panelists preferred 0.001% of Compound 3 over the control. The off-notes in this product are typically associated with soy protein.

### Example #12

### Soy Yogurt (O'Soy Organic Soy Yogurt Strawberry Flavor - 5 g protein per 4 oz serving)

| Ingredient | % (by weight) |
|---|---|
| Soy Yogurt (O'Soy Organic Soy Yogurt Strawberry Flavor) | 100 |
| Compound 1 | 0.002 |
| Soy Yogurt (O'Soy Organic Soy Yogurt Strawberry Flavor) | Balance to 100 |

| Ingredient | % (by weight) |
|---|---|
| Compound 3 | 0.001 |
| Soy Yogurt (O'Soy Organic Soy Yogurt Strawberry Flavor) | Balance to 100 |
| Compound 1 | 0.002 |
| Compound 3 | 0.001 |
| Soy Yogurt (O'Soy Organic Soy Yogurt Strawberry Flavor) | Balance to 100 |

*Bench taste findings:* Panelists preferred the blend of Compound 1 @ 0.002% and Compound 3 @ 0.001% over Compound 1 @ 0.002% or Compound 3 @ 0.001% alone. The off-notes in this product are typically associated with soy protein.

### Example #13

### Fortified Mayonnaise (Smart Balance Omega Plus Light Mayonnaise Dressing - omega-3 (500 mg per 15 g serving) and plant sterols (100 mg per 15 g serving)

| Ingredient | % (by weight) |
|---|---|
| Fortified Mayonnaise (Smart Balance Omega Plus Light Mayonnaise Dressing) | 100 |

| Ingredient | % (by weight) |
|---|---|
| Compound 1 | 0.00025 |
| Fortified Mayonnaise (Smart Balance Omega Plus Light Mayonnaise Dressing) | Balance to 100 |

| Ingredient | % (by weight) |
|---|---|
| Compound 1 | 0.00025 |
| Fortified Mayonnaise (Smart Balance Omega Plus Light Mayonnaise Dressing) | Balance to 100 |

*Bench taste findings:* Panelists preferred 0.00025% of Compound 1 over the control. The off-notes in this product are typically associated with omega-3 and plant sterols.

### Example #14

### Fortified Mayonnaise (Smart Balance Omega Plus Light Mayonnaise Dressing - omega-3 (500 mg per 15 g serving) and plant sterols (100 mg per 15 g serving)

| Ingredient | % (by weight) |
|---|---|
| Fortified Mayonnaise (Smart Balance Omega Plus Light Mayonnaise Dressing) | 100 |

| Ingredient | % (by weight) |
|---|---|
| Compound 2 | 0.0005 |
| Fortified Mayonnaise (Smart Balance Omega Plus Light Mayonnaise Dressing) | Balance to 100 |

| Ingredient | % (by weight) |
|---|---|
| Compound 2 | 0.001 |
| Fortified Mayonnaise (Smart Balance Omega Plus Light Mayonnaise Dressing) | Balance to 100 |

*Bench taste findings:* Panelists preferred 0.0005% of Compound 2 over the control. The off-notes in this product are typically associated with omega-3 and plant sterols.

### Example #15

### Fortified Mayonnaise (Smart Balance Omega Plus Light Mayonnaise Dressing - omega-3 (500 mg per 15 g serving) and plant sterols (100 mg per 15 g serving)

| Ingredient | % (by weight) |
|---|---|
| Fortified Mayonnaise (Smart Balance Omega Plus Light Mayonnaise Dressing) | 100 |

| Ingredient | % (by weight) |
|---|---|
| Compound 3 | 0.0005 |
| Fortified Mayonnaise (Smart Balance Omega Plus Light Mayonnaise Dressing) | Balance to 100 |

| Ingredient | % (by weight) |
|---|---|
| Compound 3 | 0.001 |
| Fortified Mayonnaise (Smart Balance Omega Plus Light Mayonnaise Dressing) | Balance to 100 |

*Bench taste findings:* Panelists preferred 0.001% of Compound 3 over the control. The off-notes in this product are typically associated with omega-3 and plant sterols.

### Example #16

### Fortified Mayonnaise (Smart Balance Omega Plus Light Mayonnaise Dressing - omega-3 (500 mg per 15 g serving) and plant sterols (100 mg per 15 g serving)

| Ingredient | % (by weight) |
|---|---|
| Fortified Mayonnaise (Smart Balance Omega Plus Light Mayonnaise Dressing) | 100 |

| Ingredient | % (by weight) |
|---|---|
| Compound 1 | 0.00025 |
| Fortified Mayonnaise (Smart Balance Omega Plus Light Mayonnaise Dressing) | Balance to 100 |

| Ingredient | % (by weight) |
|---|---|
| Compound 2 | 0.0005 |
| Fortified Mayonnaise (Smart Balance Omega Plus Light Mayonnaise Dressing) | Balance to 100 |

| Ingredient | % (by weight) |
|---|---|
| Compound 1 | 0.00025 |
| Compound 2 | 0.0005 |
| Fortified Mayonnaise (Smart Balance Omega Plus Light Mayonnaise Dressing) | Balance to 100 |

*Bench taste findings:* Panelists preferred the blend of Compound 1 @ 0.00025% and Compound 2 @ 0.0005% over Compound 1 @ 0.00025% and Compound 2 @ 0.0005% alone. The off-notes in this product are typically associated with omega-3 and plant sterols.

### Example #17

### Fortified Mayonnaise (Smart Balance Omega Plus Light Mayonnaise Dressing - omega-3 (500 mg per 15 g serving) and plant sterols (100 mg per 15 g serving)

| Ingredient | % (by weight) |
|---|---|
| Fortified Mayonnaise (Smart Balance Omega Plus Light Mayonnaise Dressing) | 100 |

| Ingredient | % (by weight) |
|---|---|
| Compound 1 | 0.00025 |
| Fortified Mayonnaise (Smart Balance Omega Plus Light Mayonnaise Dressing) | Balance to 100 |

| Ingredient | % (by weight) |
|---|---|
| Compound 3 | 0.001 |
| Fortified Mayonnaise (Smart Balance Omega Plus Light Mayonnaise Dressing) | Balance to 100 |

| Ingredient | % (by weight) |
|---|---|
| Compound 1 | 0.00025 |

| Compound 3 | 0.001 |
|---|---|
| Fortified Mayonnaise (Smart Balance Omega Plus Light Mayonnaise Dressing) | Balance to 100 |

*Bench taste findings:* Panelists preferred either Compound 1 @ 0.00025% OR Compound 3 @ 0.001% over the control. The blend of Compound 1 @ 0.00025% and Compound 3 @ 0.001% was preferred over the control but not necessarily over the individual ingredients (not enough tasters to determine statistics). The off-notes in this product are typically associated with omega-3 and plant sterols.

### Example #18

### Over-the-Counter (OTC) Syrup (Kroger Daytime Cold/Flu Relief (Acetaminophen 325 mg per 15 mL; Dextromethorphan HBr 10 mg per 15 mL; Phenylephrine HCl 5 mg per 15 mL))

| Ingredient | % (by weight) |
|---|---|
| OTC Syrup (Kroger Daytime Cold/Flu Relief) | 100 |

| Ingredient | % (by weight) |
|---|---|
| Compound 1 | 0.002 |
| OTC Syrup (Kroger Daytime Cold/Flu Relief) | Balance to 100 |

| Ingredient | % (by weight) |
|---|---|
| Compound 1 | 0.003 |
| OTC Syrup (Kroger Daytime Cold/Flu Relief) | Balance to 100 |

*Bench taste findings:* Panelists preferred 0.003% of Compound 1 over the control. The off-notes in this product are typically associated with OTC syrups.

### Example #19

### Over-the-Counter (OTC) Syrup (Kroger Daytime Cold/Flu Relief (Acetaminophen 325 mg per 15 mL; Dextromethorphan HBr 10 mg per 15 mL; Phenylephrine HCl 5 mg per 15 mL))

| Ingredient | % (by weight) |
|---|---|
| OTC Syrup (Kroger Daytime Cold/Flu Relief) | 100 |

| Ingredient | % (by weight) |
|---|---|
| Compound 2 | 0.002 |
| OTC Syrup (Kroger Daytime Cold/Flu Relief) | Balance to 100 |

| Ingredient | % (by weight) |
|---|---|
| Compound 2 | 0.003 |
| OTC Syrup (Kroger Daytime Cold/Flu Relief) | Balance to 100 |

*Bench taste findings:* Panelists preferred 0.003% of Compound 2 over the control. The off-notes in this product are typically associated with OTC syrups.

### Example #20

### Over-the-Counter (OTC) Syrup (Kroger Daytime Cold/Flu Relief (Acetaminophen 325 mg per 15 mL; Dextromethorphan HBr 10 mg per 15 mL; Phenylephrine HCl 5 mg per 15 mL))

| Ingredient | % (by weight) |
|---|---|
| OTC Syrup (Kroger Daytime Cold/Flu Relief) | 100 |

| Ingredient | % (by weight) |
|---|---|
| Compound 3 | 0.001 |
| OTC Syrup (Kroger Daytime Cold/Flu Relief) | Balance to 100 |

| Ingredient | % (by weight) |
|---|---|
| Compound 2 | 0.002 |
| OTC Syrup (Kroger Daytime Cold/Flu Relief) | Balance to 100 |

*Bench taste findings:* Panelists preferred 0.002% of Compound 3 over the control. The off-notes in this product are typically associated with OTC syrups.

### Example #21

### Over-the-Counter (OTC) Syrup (Kroger Daytime Cold/Flu Relief (Acetaminophen 325 mg per 15 mL; Dextromethorphan HBr 10 mg per 15 mL; Phenylephrine HCl 5 mg per 15 mL))

| Ingredient | % (by weight) |
|---|---|
| OTC Syrup (Kroger Daytime Cold/Flu Relief) | 100 |

| Ingredient | % (by weight) |
|---|---|
| Compound 1 | 0.003 |
| OTC Syrup (Kroger Daytime Cold/Flu Relief) | Balance to 100 |

| Ingredient | % (by weight) |
|---|---|
| Compound 3 | 0.002 |
| OTC Syrup (Kroger Daytime Cold/Flu | Balance to 100 |
| Relief) | |

| Ingredient | % (by weight) |
|---|---|
| Compound 1 | 0.003 |
| Compound 3 | 0.002 |
| OTC Syrup (Kroger Daytime Cold/Flu Relief) | Balance to 100 |

*Bench taste findings:* Panelists preferred the blend of Compound 1 @ 0.003% and Compound 3 @ 0.002% over Compound 1 @ 0.003% and Compound 3 @ 0.002% alone. The off-notes in this product are typically associated with OTC syrups.

### Example #22

### Over-the-Counter (OTC) Syrup (Kroger Daytime Cold/Flu Relief (Acetaminophen 325 mg per 15 mL; Dextromethorphan HBr 10 mg per 15 mL; Phenylephrine HCl 5 mg per 15 mL))

| Ingredient | % (by weight) |
|---|---|
| OTC Syrup (Kroger Daytime Cold/Flu Relief) | 100 |

| Ingredient | % (by weight) |
|---|---|
| Compound 1 | 0.003 |
| OTC Syrup (Kroger Daytime ColdlFlu Relief) | Balance to 100 |

| Ingredient | % (by weight) |
|---|---|
| Compound 2 | 0.003 |
| OTC Syrup (Kroger Daytime Cold/Flu Relief) | Balance to 100 |

| Ingredient | % (by weight) |
|---|---|
| Compound 1 | 0.003 |
| Compound 2 | 0.003 |
| OTC Syrup (Kroger Daytime Cold/lu Relief) | Balance to 100 |

*Bench taste findings:* Panelists preferred either Compound 1 @ 0.003% OR Compound 2 @ 0.003% over the control. The blend of Compound 1 @ 0.003% and Compound 2 @ 0.003% was preferred over the control but not necessarily over the individual ingredients (not enough tasters to determine statistics). The off-notes in this product are typically associated with OTC syrups.

### Example #23

### Proteinaceous Supplement (GNC Pro Performance 100% Whey Protein)

| Ingredient | % (by weight) |
|---|---|
| Proteinaceous Supplement (GNC Pro Performance 100% Whey Protein) | 100 |

| Ingredient | % (by weight) |
|---|---|
| Compound 1 | 0.00075 |
| Proteinaceous Supplement (GNC Pro Performance 100% Whey Protein) | Balance to 100 |

| Ingredient | % (by weight) |
|---|---|
| Compound 1 | 0.0015 |
| Proteinaceous Supplement (GNC Pro Performance 100% Whey Protein) | Balance to 100 |

| Ingredient | % (by weight) |
|---|---|
| Compound 1 | 0.003 |
| Proteinaceous Supplement (GNC Pro Performance 100% Whey Protein) | Balance to 100 |

*Bench taste findings:* Panelists preferred Compound 1 @ 0.003% over the control. The off-notes in this product are typically associated with whey protein.

### Example #24

### Proteinaceous Supplement (GNC Pro Performance 100% Whey Protein)

| Ingredient | % (by weight) |
|---|---|
| Proteinaceous Supplement (GNC Pro Performance 100% Whey Protein) | 100 |

| Ingredient | % (by weight) |
|---|---|
| Compound 2 | 0.00075 |
| Proteinaceous Supplement (GNC Pro Performance 100% Whey Protein) | Balance to 100 |

| Ingredient | % (by weight) |
|---|---|
| Compound 2 | 0.0015 |
| Proteinaceous Supplement (GNC Pro Performance 100% Whey Protein) | Balance to 100 |

| Ingredient | % (by weight) |
|---|---|
| Compound 2 | 0.0015 |
| Proteinaceous Supplement (GNC Pro Performance 100% Whey Protein) | Balance to 100 |

*Bench taste findings:* Panelists preferred Compound 2 @ 0.0015% over the control. The off-notes in this product are typically associated with whey protein.

### Example #25

### Proteinaceous Supplement (GNC Pro Performance 100% Whey Protein)

| Ingredient | % (by weight) |
|---|---|
| Proteinaceous Supplement (GNC Pro Performance 100% Whey Protein) | 100 |

| Ingredient | % (by weight) |
|---|---|
| Compound 3 | 0.00075 |
| Proteinaceous Supplement (GNC Pro Performance 100% Whey Protein) | Balance to 100 |

| Ingredient | % (by weight) |
|---|---|
| Compound 3 | 0.0015 |
| Proteinaceous Supplement (GNC Pro Performance 100% Whey Protein) | Balance to 100 |

| Ingredient | % (by weight) |
|---|---|
| Compound 3 | 0.0015 |
| Proteinaceous Supplement (GNC Pro Performance 100% Whey Protein) | Balance to 100 |

*Bench taste findings:* Panelists preferred Compound 3 @ 0.0015% over the control. The off-notes in this product are typically associated with whey protein.

### Example #26

### Proteinaceous Supplement (GNC Pro Performance 100% Whey Protein)

| Ingredient | % (by weight) |
|---|---|
| Proteinaceous Supplement (GNC Pro Performance 100% Whey Protein) | 100 |

| Ingredient | % (by weight) |
|---|---|
| Compound 1 | 0.003 |
| Proteinaceous Supplement (GNC Pro Performance 100% Whey Protein) | Balance to 100 |

| Ingredient | % (by weight) |
|---|---|
| Compound 2 | 0.0015 |
| Proteinaceous Supplement (GNC Pro Performance 100% Whey Protein) | Balance to 100 |

| Ingredient | % (by weight) |
|---|---|
| Compound 1 | 0.003 |
| Compound 2 | 0.0015 |
| Proteinaceous Supplement (GNC Pro Performance 100% Whey Protein) | Balance to 100 |

*Bench taste findings:* Panelists preferred the blend of Compound 1 @ 0.003% and Compound 2 @ 0.0015% over Compound 2 @ 0.0015% alone. The off-notes in this product are typically associated with whey protein.

### Example #27

### Proteinaceous Supplement (GNC Pro Performance 100% Whey Protein)

| Ingredient | % (by weight) |
|---|---|
| Proteinaceous Supplement (GNC Pro | 100 |
| Performance 100% Whey Protein) | |

| Ingredient | % (by weight) |
|---|---|
| Compound 1 | 0.003 |
| Proteinaceous Supplement (GNC Pro Performance 100% Whey Protein) | Balance to 100 |

| Ingredient | % (by weight) |
|---|---|
| Compound 3 | 0.0015 |
| Proteinaceous Supplement (GNC Pro Performance 100% Whey Protein) | Balance to 100 |

| Ingredient | % (by wight) |
|---|---|
| Compound 1 | 0.003 |
| Compound 3 | 0.003 |
| Proteinaceous Supplement (GNC Pro Performance 100% Whey Protein) | Balance to 100 |

*Bench taste findings:* Panelists preferred the blend of Compound 1 @ 0.003% and Compound 3 @ 0.0015% over Compound 1 @ 0.003% and Compound 3 @ 0.0015% alone. The off-notes in this product are typically associated with whey protein.

### Example #28

### Cocoa (dilute 10% in water to consume) (Kroger Cocoa Unsweetened)

| Ingredient | % (by weight) |
|---|---|
| Cocoa (dilute 10% in water) (Kroger Cocoa Unsweetened) | 100 |

| Ingredient | % (by weight) |
|---|---|
| Compound 1 | 0.002 |
| Cocoa (dilute 10% in water) (Kroger Cocoa Unsweetened) | Balance to 100 |

| Ingredient | % (by weight) |
|---|---|
| Compound 1 | 0.003 |
| Cocoa (dilute 10% in water) (Kroger Cocoa Unsweetened) | Balance to 100 |

*Bench taste findings:* Panelists preferred Compound 1 @ 0.002% over the control. The off-notes in this product are typically associated with cocoa (unsweetened).

### Example #29

### Cocoa (dilute 10% in water to consume) (Kroger Cocoa Unsweetened)

| Ingredient | % (by weight) |
|---|---|
| Cocoa (dilute 10% in water) (Kroger Cocoa Unsweetened) | 100 |

| Ingredient | % (by weight) |
|---|---|
| Compound 3 | 0.001 |
| Cocoa (dilute 10% in water) (Kroger Cocoa Unsweetened) | Balance to 100 |

| Ingredient | % (by weight) |
|---|---|
| Compound 3 | 0.002 |
| Cocoa (dilute 10% in water) (Kroger Cocoa Unsweetened) | Balance to 100 |

*Bench taste findings:* Panelists preferred Compound 3 @ 0.001% over the control. The off-notes in this product are typically associated with cocoa (unsweetened).

### Example #30

### Cocoa (dilute 10% in water to consume) (Kroger Cocoa Unsweetened)

| Ingredient | % (by weight) |
|---|---|
| Cocoa (dilute 10% in water) (Kroger Cocoa Unsweetened) | 100 |

| Ingredient | % (by weight) |
|---|---|
| Compound 2 | 0.001 |
| Cocoa (dilute 10% in water) (Kroger Cocoa Unsweetened) | Balance to 100 |

| Ingredient | % (by weight) |
|---|---|
| Compound 3 | 0.002 |
| Cocoa (dilute 10% in water) (Kroger Cocoa Unsweetened) | Balance to 100 |

*Bench taste findings:* Panelists preferred Compound 2 @ 0.002% over the control. The off-notes in this product are typically associated with cocoa (unsweetened).

### Example #31

### Cocoa (dilute 10% in water to consume) (Kroger Cocoa Unsweetened)

| Ingredient | % (by weight) |
|---|---|
| Cocoa (dilute 10% in water) (Kroger Cocoa Unsweetened) | 100 |

| Ingredient | % (by weight) |
|---|---|
| Compound 1 | 0.002 |
| Cocoa (dilute 10% in water) (Kroger Cocoa Unsweetened) | Balance to 100 |

| Ingredient | % (by weight) |
|---|---|
| Compound 2 | 0.002 |
| Cocoa (dilute 10% in water) (Kroger Cocoa Unsweetened) | Balance to 100 |

| Ingredient | % (by weight) |
|---|---|
| Compound 1 | 0.002 |
| Compound 2 | 0.002 |
| Cocoa (dilute 10% in water) (Kroger Cocoa Unsweetened) | Balance to 100 |

*Bench taste findings:* Panelists preferred either Compound 1 @ 0.002% OR Compound 2 @ 0.002% over the control. The blend of Compound 1 @ 0.003% and Compound 2 @ 0.003% was NOT preferred over the control. The off-notes in this product are typically associated with cocoa (unsweetened).

### Example #32

### Cocoa (dilute 10% in water to consume) (Kroger Cocoa Unsweetened)

| Ingredient | % (by weight) |
|---|---|
| Cocoa (dilute 10% in water) (Kroger Cocoa Unsweetened) | 100 |

| Ingredient | % (by weight) |
|---|---|
| Compound 1 | 0.002 |
| Cocoa (dilute 10% in water) (Kroger Cocoa Unsweetened) | Balance to 100 |

| Ingredient | % (by weight) |
|---|---|
| Compound 3 | 0.001 |
| Cocoa (dilute 10% in water) (Kroger Cocoa Unsweetened) | Balance to 100 |

| Ingredient | % (by weight) |
|---|---|
| Compound 1 | 0.002 |
| Compound 3 | 0.001 |
| Cocoa (dilute 10% in water) (Kroger Cocoa Unsweetened) | Balance to 100 |

*Bench taste findings:* Panelists preferred either Compound 1 @ 0.002% OR Compound 3 @ 0.001% over the control. The blend of Compound 1 @ 0.002% and Compound 3 @ 0.001% was NOT preferred over the control. The off-notes in this product are typically associated with cocoa (unsweetened).

Although the embodiments have been described in detail through the above description and the preceding examples, these examples are for the purpose of illustration only and it is understood that variations and modifications can be made by one skilled in the art without departing from the scope of the disclosure. It should be understood that the embodiments described above are not only in the alternative, but can be combined.

## Claims

1. A method of masking an off-note in an orally-receivable or -ingestible composition, comprising the addition thereto of an off-note masking quantity of 1-(2-hydroxyphenyl)-3-(pyridin-4-yl)propane-1-one or a salt thereof, and a compound selected from the group consisting of 4-[2,2,3-trimethyl-cyclopentyl]-butanoic acid, 2-[(3-ethyl-4,8-dimethyl-2-oxo-2H-chromen-7-yl)oxy]propanoic acid, and salts thereof.

2. An off-note-masked orally-receivable or -ingestible composition comprising an off-note masking quantity of a masking composition comprising 1-(2-hydroxyphenyl)-3-(pyridin-4-yl)propane-1-one and at least one of 4-[2,2,3-trimethyl-cyclopentyl]-butanoic acid or 2-[(3-ethyl-4,8-dimethyl-2-oxo-2H-chromen-7-yl)oxy]propanoic acid or salts thereof.

3. An off-note-masking composition consisting of 1-(2-hydroxyphenyl)-3-(pyridin-4-yl)propane-1-one and at least one of 4-[2,2,3-trimethyl-cyclopentyl]-butanoic acid or 2-[(3-ethyl-4,8-dimethyl-2-oxo-2H-chromen-7-yl)oxy]propanoic acid or salts thereof.

4. An off-note-masking composition according to claim 3, consisting of 1-(2-hydroxyphenyl)-3-(pyridin-4-yl)propane-1-one and 4-[2,2,3-trimethyl-cyclopentyl]-butanoic acid, or salts thereof.

5. An off-note-masking composition according to claim 3, consisting of 1-(2-hydroxyphenyl)-3-(pyridin-4-yl)propane-1-one and 2-[(3-ethyl-4,8-dimethyl-2-oxo-2H-chromen-7-yl)oxy]propanoic acid, or salts thereof.

6. An off-note-masking composition according to claim 3, consisting of 1-(2-hydroxyphenyl)-3-(pyridin-4-yl)propane-1-one, 4-[2,2,3-trimethyl-cyclopentyl]-butanoic acid and 2-[(3-ethyl-4,8-dimethyl-2-oxo-2H-chromen-7-yl)oxy]propanoic acid, or salts thereof.

## Patentansprüche

1. Verfahren zur Maskierung einer Fehlnote in einer oral auf- oder einnehmbaren Zusammensetzung, bei dem man dieser eine fehlnotenmaskierende Menge von 1-(2-Hydroxyphenyl)-3-(pyridin-4-yl)propan-1-on oder einem Salz davon und einer Verbindung aus der Gruppe bestehend aus 4-[2,2,3-Trimethylcyclo-pentyl]butansäure, 2-[(3-Ethyl-4,8-dimethyl-2-oxo-2H-chromen-7-yl)oxy]propansäure und Salzen davon zusetzt.

2. Fehlnotenmaskierte oral auf- oder einnehmbare Zusammensetzung, umfassend eine fehlnotenmaskierende Menge einer Maskierungszusammensetzung, die 1-(2-Hydroxyphenyl)-3-(pyridin-4-yl)-propan-1-on und mindestens eine der Verbindungen 4-[2,2,3-Trimethylcyclopentyl]butansäure oder 2-[(3-Ethyl-4,8-dimethyl-2-oxo-2H-chromen-7-yl)oxy]-propansäure oder Salze davon umfasst.

3. Fehlnotenmaskierende Zusammensetzung, bestehend aus 1-(2-Hydroxyphenyl)-3-(pyridin-4-yl)propan-1-on und mindestens einer der Verbindungen 4-[2,2,3-Trimethylcyclopentyl]butansäure oder 2-[(3-Ethyl-4,8-dimethyl-2-oxo-2H-chromen-7-yl)oxy]propansäure und/oder Salzen davon.

4. Fehlnotenmaskierende Zusammensetzung nach Anspruch 3, bestehend aus 1-(2-Hydroxyphenyl)-3-(pyridin-4-yl)propan-1-on und 4-[2,2,3-Trimethylcyclopentyl]-butansäure oder Salzen davon.

5. Fehlnotenmaskierende Zusammensetzung nach Anspruch 3, bestehend aus 1-(2-Hydroxyphenyl)-3-(pyridin-4-yl)propan-1-on und 2-[(3-Ethyl-4,8-dimethyl-2-oxo-2H-chromen-7-yl)oxy]propansäure oder Salzen davon.

6. Fehlnotenmaskierende Zusammensetzung nach Anspruch 3, bestehend aus 1-(2-Hydroxyphenyl)-3-(pyridin-4-yl)propan-1-on, 4-[2,2,3-Trimethylcyclopentyl]-butansäure und 2-[(3-Ethyl-4,8-dimethyl-2-oxo-2H-chromen-7-yl)oxy]propansäure oder Salzen davon.

## Revendications

1. Méthode de masquage d'une note atypique dans une composition recevable ou ingérable par voie orale, comprenant l'addition à celle-ci d'une quantité de masquage de note atypique de 1-(2-hydroxyphényl)-3-(pyridin-4-yl)propane-1-one ou d'un sel de celle-ci, et d'un composé choisi dans le groupe constitué par l'acide 4-[2,2,3-triméthylcyclopentyl]butanoïque, l'acide 2-[(3-éthyl-4,8-diméthyl-2-oxo-2H-chromén-7-yl)oxy]propanoïque, et des sels de ceux-ci.

2. Composition recevable ou ingérable par voie orale, à masquage de note atypique, comprenant une quantité de masquage de note atypique d'une composition de masquage comprenant de la 1-(2-hydroxyphényl)-3-(pyridin-4-yl)propane-1-one, et d'au moins un parmi l'acide 4-[2,2,3-triméthylcyclopentyl]butanoïque ou l'acide 2-[(3-éthyl-4,8-diméthyl-2-oxo-2H-chromén-7-yl)oxy]-propanoïque, ou des sels de ceux-ci.

3. Composition de masquage de note atypique, constituée de 1-(2-hydroxyphényl)-3-(pyridin-4-yl)-propane-1-one, et au moins un parmi l'acide 4-[2,2,3-triméthylcyclopentyl]butanoïque ou l'acide 2-[(3-éthyl-4,8-diméthyl-2-oxo-2H-chromén-7-yl)oxy]propanoïque, ou de sels de ceux-ci.

4. Composition de masquage de note atypique selon la revendication 3, constituée de 1-(2-hydroxyphényl)-3-(pyridin-4-yl)propane-1-one, et d'acide 4-[2,2,3-triméthylcyclopentyl]butanoïque, ou de sels de ceux-ci.

5. Composition de masquage de note atypique selon la revendication 3, constituée de 1-(2-hydroxyphényl)-3-(pyridin-4-yl)propane-1-one, et d'acide 2-[(3-éthyl-4,8-diméthyl-2-oxo-2H-chromén-7-yl)oxy]propanoïque, ou de sels de ceux-ci.

6. Composition de masquage de note atypique selon la revendication 3, constituée de 1-(2-hydroxyphényl)-3-(pyridin-4-yl)propane-1-one, d'acide 4-[2,2,3-triméthylcyclopentyl]butanoïque et d'acide 2-[(3-éthyl-4,8-diméthyl-2-oxo-2H-chromén-7-yl)oxy]propanoïque, ou de sels de ceux-ci.
